# EUROPÄISCHE PATENTSCHRIFT

(11) **EP 2 515 922 B1**
(45) Veröffentlichungstag und Bekanntmachung des Hinweises auf die Patenterteilung: **02.10.2013**
(21) Anmeldenummer: 10800741.0
(22) Anmeldetag: 22.12.2010
(51) Int. Cl.: A61K 36/53, A61P 25/28

(54) **PFLANZENEXTRAKTE ZUR BEHANDLUNG VON NEURODEGENERATIVEN ERKRANKUNGEN**
PLANT EXTRACT FOR TREATING NEURODEGENERATIVE ILLNESSES
EXTRAITS DE PLANTES DESTINÉS AU TRAITEMENT DE MALADIES NEURODÉGÉNÉRATIVES

(30) Priorität: 23.12.2009 EP 09180627
(43) Veröffentlichungstag der Anmeldung: 31.10.2012
(73) Patentinhaber: Finzelberg GmbH & Co. KG, 56626 Andernach (DE)
(72) Erfinder: WALBROEL, Bernd, 53639 Königswinter (DE); FEISTEL, Björn, 56626 Andernach (DE); PAHNKE, Jens, 18147 Rostock (DE)
(74) Vertreter: von Kreisler Selting Werner
(86) Internationale Anmeldenummer: PCT/EP2010/070531
(87) Internationale Veröffentlichungsnummer: WO 2011/076867

(56) Entgegenhaltungen:
- DE-A1-102004 032 165
- RAINER KNÖRLE ET AL: "Extrakte aus Sideritis ssp. (griechischer Bergtee): Innovative zentral aktive Pflanzenextrakte mit breitem Wirkprofil", INTERNET CITATION, 1. Oktober 2009 (2009-10-01), Seiten 1-15, XP002591208, Gefunden im Internet: URL:http://ibam.de/pics/Poster-Wolnzach-20 09.pdf [gefunden am 2010-07-08]

## Beschreibung

Die vorliegende Erfindung betrifft Pflanzenextrakte und ihre Verwendung.

Neurodegenerative Erkrankungen mit Proteinablagerungen umfassen ein breites Spektrum von Krankheitsbildern. Diese sind gekennzeichnet durch pathologisch abnorme, globale oder lokale Konzentrationen von Proteinen oder Proteinfragmenten (Peptide) und hierdurch ausgelöste Ausfällungen bzw. Aggregationen, welche gegebenenfalls durch vorherige Dimerisierung oder Oligomerisierung zu geordneten Strukturen (z.B. Fibrillen) führen und welche sogar kristalline Proteinstrukturen bilden können (z.B. ß-Amyloid in Alzheimer Plaques).

Unter anderem verbergen sich hinter dieser Pathologie Krankheiten wie Chorea Huntington, bei welcher Huntingtin akkumuliert und sich in Kernregionen des Gehirnes ablagert. Die Spinozerebelläre Ataxie Typ 2 (SCA2) ist eine neurodegenerative Erkrankung, bei der das Protein Ataxin-2 verantwortlich gemacht wird und in agglomerierter Form nachgewiesen wurde. Beim der Lewy-Körperchen-Demenz (LBD) ist die agglomerierte Form des Proteins α-Synuclein pathophysiologisch festzustellen, welches auch die Krankheitsbilder wie Morbus Parkinson und Multisystematrophie (MSA) in der Gruppe der "Synucleinopathien" zusammenführt. Eine weitere Gruppe von neurodegenerativen Erkrankungen bilden die Tauopathien, bei denen sich das Zytoskelettprotein Tau-Protein zu fibrillären Strukturen (PHFs oder Tangles) zusammenlagert.

Eine große Gruppe solcher Proteinausfällungen beobachtet man bei Amyloidosen. Hierbei treten große Mengen abnorm veränderter Proteine auf, welche sich als unlösliche kleine Fasern (Fibrillen) ablagern. Im Falle der Amyloidosen liegt beispielsweise eine Störung der Faltung eines normalerweise löslichen Proteins zu Grunde. Diese Proteine lagern sich durch abnorme Konzentrationen zu größeren ß-Faltblattstrukturen zusammen und bilden schlussendlich Fibrillen.

Da die Fibrillen gegenüber körpereigenen Abwehrmechanismen (Phagozytose und Proteolyse) relativ resistent sind, können sie aus den befallenen Organen nicht vollständig entfernt werden. Diese Ablagerungen zerstören dann die Architektur der Organe und führen dadurch zu Fehlfunktionen, bis hin zum Totalausfall. Amyloidosen sind ein krankhafter Ablagerungsprozess, der von unterschiedlichen Stoffwechseldefekten ausgelöst werden kann und je nach betroffenem Organ zu unterschiedlichen chronischen Veränderungen oder Krankheiten führt.

Eine spezielle Ausprägung der Amyloidose stellt die Altersamyloidose dar, welche hauptsächlich Herz und Gehirn betrifft. Heutzutage wird diese Form immunhistochemisch als AS-Amyloidose beschrieben. Hierunter versteht man alle Amyloidproteine die eine senile Amyloidose zur Folge haben. Obwohl vielfach die auslösenden Proteine noch nicht charakterisiert sind, wird ein gehäuftes Auftreten mit dem Morbus Alzheimer in Verbindung gebracht.

Eine weitere periphere degenerative Erkrankungen mit Proteinablagerung ist z.B. die Einschlusskörpermyositis (Inclusion Body Myositis). Dort finden sich abgelagerte Moleküle/Peptide innerhalb von Muskelfasern, die mit degenerativen Organprozessen auch anderorts, wie z.B. ß-Amyloiden und Prion Protein, in Verbindung gebracht werden. Letztere werden unter anderem als ursächlich für den entzündliche Muskelprozess bei der IBM betrachtet.

Der Morbus Alzheimer, die Alzheimer-Krankheit (engl. Alzheimer Disease) oder auch bezeichnet als Alzheimer Demenz, international abgekürzt unter AD, ist eine neurodegenerative Erkrankung, welche in ihrer häufigsten Form bei Personen oberhalb von 65 Jahren auftritt und im Jahre 2007 für weltweit etwa 29 Millionen Demenzerkrankungen verantwortlich gemacht wird. [R. Brookmeyer u. a.: Forecasting the global burden of Alzheimer's disease. In: Alzheimer's and Dementia. 3/2007, Band 3, Seiten 186-191]. Mit Fortschreiten der Erkrankung verschlechtern sich die kognitiven Fähigkeiten, was bis zu Verhaltensauffälligkeiten und Wesensveränderungen führen kann. Im Gehirn der Betroffenen können sogenannte Plaques gefunden werden, welche aus aggregierten ß-Amyloid-Peptiden (Aß) bestehen. Bislang konnte noch keine eindeutige Ursache für die sporadische Variante der Erkrankung gefunden werden, jedoch werden Genmutationen auf drei verschiedenen Genen mit familiärer AD in Verbindung gebracht. Ebenso werden spezielle Allel-Genotypen des Apolipoprotein E (ApoE), speziell des ApoE4, für eine Risikoerhöhung, an einer AD zu erkranken, verantwortlich gemacht. [Corder, E.H. et al (1993): Gene dose of apolipoprotein E type 4 allele and the risk of Alzheimer's disease in late onset families. In: Science. 261 (5123):921-923][ Bu, G. (2009): Apolipoprotein E and its receptors in Alzheimer's disease: pathways, pathogenesis and therapy. In: Nat. Rev. Neurosci. 10(5):333-344.]. Auch auf Grund der demographischen Entwicklung mit zunehmender Alterung der Bevölkerung werden in den kommenden Jahrzehnten immer mehr Menschen an AD erkranken. Die Alzheimer-Prävalenz steigt von 2% bei 65-Jährigen auf 20% bei 85-Jährigen. Prognosen sehen für das Jahr 2050 eine Prävalenz auf über 100 Millionen Patienten vorher. Diese Patientenmengen stellen damit eine erhebliche Belastung für das Allgemeinwesen dar. Eine Verzögerung in der Pathogenese um ca. 5 Jahre könnte sowohl die individuelle Leidensgeschichte bis zum natürlichen Tod verschieben, als auch gemeinwirtschaftlich die zu erwartenden Kosten deutlich vermindern.

Betrachtet man den Pathomechanismus näher, so findet man im Gehirn von Alzheimer-Patienten sogenannte senile Plaques und fibrilläre Tangles (NFT). Die Proteinablagerungen der Plaques bestehen im Wesentlichen aus Amyloid-ß-Peptiden. Die intrazellulär gelegenen Neurofibrillenbündel bestehen aus dem Tau-Protein. Dieses aggregiert zu Fibrillen, wenn es stärker als normal phosphoryliert, d.h. mit Phosphorsäureresten besetzt ist ("Hyperphosphorylierung"). Es ist ungeklärt, ob diese Tau-Phosphorylierung sekundärer Natur oder krankheitsauslösend ist. Im Krankheitsverlauf nimmt die Hirnmasse durch das Absterben von Neuronen ab; man spricht dann von einer Hirnatrophie. Außerdem wird der Botenstoff Acetylcholin nicht mehr in ausreichenden Mengen produziert, was zu einer allgemeinen Leistungsschwächung des Gehirns führt.

Die Aß-Peptide entstehen aus einem Vorläuferprotein, dem Amyloid-Precursor-Protein (APP), bei dem es sich um ein integrales Membranprotein handelt. Es handelt sich um ein Typ I - Transmembranprotein: Sein großer Amino-Terminus befindet sich auf der Zellaußenseite, während ein kleinerer Carboxyl-Terminus innerhalb der Zelle zu finden ist. APP wird von proteinspaltenden Enzymen, den sogenannten Sekretasen (α-Sekretase, ß-Sekretase und γ-Sekretase) gespalten, wodurch es zur Freisetzung des Aß-Peptids aus dem Vorläuferprotein kommen kann. Grundsätzlich gibt es zwei Wege, wie APP gespalten werden kann. Der nicht-amyloidogene Weg: APP wird durch eine α-Sekretase geschnitten. Dieser Schnitt findet innerhalb des Teils von APP statt, der Aß enthält. Dadurch wird die Bildung von Aß verhindert. Es kommt zur Freisetzung eines großen extrazellulären Anteils, dessen Funktion nicht endgültig geklärt ist. Der amyloidogene Weg: APP wird zuerst von der ß-Sekretase geschnitten und nachfolgend von der γ-Sekretase. Dieser Schnitt, der innerhalb der Transmembrandomäne erfolgt, führt zur Freisetzung von Aß. Beide Vorgänge können parallel in Nervenzellen stattfinden. Die durch ß- und γ-Sekretase gebildeten Aß-Peptide variieren in ihrer Länge. Der Haupttyp (Aß-40) ist 40, während ein kleiner Anteil (Aß-42) 42 Aminosäuren lang ist. Die Länge des Aß ist von zentraler pathologischer Bedeutung, da das längere Aß-42 eine wesentlich höhere Tendenz zur Aggregation aufweist als das kleinere Aß-40.

Es gibt genetische Ursachen die zur Alzheimer-Krankheit führen. Etwa ein bis fünf Prozent der Betroffenen zeigen eine familiäre Häufung (familial alzheimer's disease (FAD)), die auf Mutationen des Präsenilin-1-Gens auf Chromosom 14, des Präsenilin-2-Gens auf Chromosom 1 oder des APP-Gens auf Chromosom 21 zurückzuführen sind. Das Down-Syndrom mit seiner dreifachen Anlage von Erbmaterial des Chromosoms 21, auf dem sich das APP-Gen befindet, erhöht ebenfalls das Risiko, an einer Demenz, evtl. der Alzheimer-Krankheit, zu erkranken, wobei der psychodiagnostische Nachweis bei Menschen mit dieser Genommutation durch eine meist schon früh vorliegende kognitive Beeinträchtigung erschwert wird.

Bei der Alzheimer-Erkrankung ist des Weiteren die Funktion der Mitochondrien gestört. Eine Blockade der Atmungskette am Komplex IV führt zu einer übermäßigen Produktion von Radikalen, die die Zelle schädigen. Ob diese Blockade eine Konsequenz der übermäßigen Aß-Produktion ist oder ob Aß als Antioxidans gegen diesen neu entstandenen oxidativen Stress übermäßig produziert wird, ist bis heute offen. Um gegebenenfalls eine radikal-getriggerte Aß-Produktion zu vermeiden, können antioxidative Extrakte eingesetzt werden.

ABC-Transporter bilden eine große Familie von Membranproteinen, die spezifische Substrate aktiv über eine Zellmembran transportieren. ABC-Transporter gehören zu den primär aktiven Transportern einerseits und zu den membranständigen ATPasen andererseits. Die ABC-Transporter-Superfamilie umfasst eine der größten bekannten Proteinfamilien, mit Mitgliedern in fast jedem Organismus, vom Bakterium über Pflanzen bis zum Säugetier. Die ABC-Transporter rückten in den letzten Jahren in das Licht des Interesses, als erkannt wurde, dass sie eine beträchtliche medizinische, industrielle und ökonomische Bedeutung haben. Jährlich sterben zehntausende Menschen an Krebs, weil die Chemotherapie aufgrund der starken Expression von ABC-Transportern im Tumorgewebe fehlgeschlagen ist. Beim Menschen können Mutationen in einem Gen, das für einen ABC-Transporter kodiert, zu verschiedenen Stoffwechselkrankheiten führen. Alle eukaryotischen ABC-Transporter sind Exporter. Einige sind sehr substratspezifisch, andere multispezifisch. ABC-Transporter transportieren u.a. auch ß-Amyloid und werden deshalb als Teil des Pathomechanismus, der zur AD führt, betrachtet [Pahnke et al. 2008: Clinico-pathological function of cerebral ABC transporters - implications for the pathogenesis of Alzheimer's disease. Current Alzheimer Research 5, 396-406., Pahnke et al. 2009: Alzheimer's disease and blood-brain barrier function - Why have anti-β-amyloid therapies failed to prevent dementia progression? NeurosciBiobehavR 33:1099-1108].

Aus dem Bereich der Phytopharmaka sind heute viele Pflanzenextrakte für ihre ZNS-Aktivität bekannt. Hierfür ist es notwendig, dass Inhaltsstoffe der Pflanzen die Blut-Hirn-Schranke überwinden. Extrakte, für die eine ZNS-Aktivität in klinischen Studien nachgewiesen werden konnten, basieren auf Pflanzen wie Ginseng, Ginkgo, Passionsblume, Johanniskraut, Baldrian, Hopfen, Lavendel, Grüntee, Salbei und anderen.

Zugelassene Therapeutika für die Behandlung von neurodegenerativen Demenzerkrankungen, hierzu zählen beispielsweise die Alzheimer Demenz (AD) und die Demenz bei Morbus Parkinson (PD), dienen derzeit nur der rein symptomatischen Behandlung. Es wird überwiegend davon ausgegangen, dass bei dementiellen Erkrankungen die klinische Symptomatik durch ein Mangel am Neurotransmitter Acetylcholin (Ach) entsteht. Daher wird therapeutisch dagegen angegangen, indem man versucht, das Enzym zu hemmen, das für den Abbau von Acetylcholin verantwortlich ist (Acetylcholinesteraseinhibitoren). Diese ACh-Hemmstoffe, wie z.B. Donepezil oder Galantamin, sind in Deutschland für die Behandlung der AD zugelassen. Die aktuellen symptomatischen Therapien sind allerdings nur begrenzt in der Lage, das Fortschreiten der Erkrankung zu verzögern, eine Linderung des klinischen Bildes ist derzeit gar nicht in Betracht zu ziehen.

Eine Überprüfung des deutschen Institutes für Qualität und Wirtschaftlichkeit im Gesundheitsweisen vom August 2009 [IQWiG-Berichte - Jahr: 2009 Nr. 67: Aktualisierungsrecherche zum Bericht A05-19A (Cholinesterasehemmer bei Alzheimer Demenz)] kommt zu dem Ergebnis, dass die drei in Deutschland zugelassenen Wirkstoffe Donepezil, Galantamin und Rivastigmin bei Patienten mit leichter oder mittelschwerer Alzheimer Demenz den Abbau kognitiver Fähigkeiten nur geringfügig verzögern können. Zudem gebe es Hinweise, dass die entsprechenden Präparate die Geschwindigkeit verlangsamen können, mit der Alzheimer Patienten die Fähigkeit verlieren, Aktivitäten des täglichen Lebens zu bewältigen. Ein Nutzen hinsichtlich der gesundheitsbezogenen Lebensqualität nicht belegt, und alle drei Wirkstoffe würden zum Teil erhebliche Nebenwirkungen aufweisen.

Extrakte aus Ginkgo biloba, welche präventiv auf dementielle Erkrankungen bzw. deren Fortschreiten wirken sollen, zeigten bislang keine Erfolge bei der Behandlung von neurodegenerativen Erkrankungen mit Proteinablagerungen. Eine aktuelle groß angelegte prospektive, doppelblinde, placebokontrollierte Studien mit mehr als 3000 eingeschlossenen Patienten konnte keine Wirkung bzgl. der Senkung der Anzahl an Neuerkrankungen oder der Verlangsamung des klinischen Verlaufes bei bereits begonnener Demenzerkrankungen zeigen [DeKosky et al.; Ginkgo biloba for Prevention of Dementia: A Randomized Controlled Trial. JAMA. 2008;300(19):2253-2262].

Pflanzenextrakte aus Johanniskraut (*Hypericum perforatum spp.*) sind bekannt als ZNS aktive Extrakte und werden bei milder bis mittelschwerer Depression als "mood lifter" eingesetzt. Hier werden so genannte Gesamt-Extrakte aus alkoholisch-wässrigen Lösungen als *active pharmaceutical ingredient* (API) verwendet. Silva et al. [Neurotoxicity Research, 2004, Vol. 6(2), pp119-130] beschreibt einen neuroprotektiven Effekt eines Hypericum-Extraktes auf ß-Amyloid-induzierter Neurotoxizität. Der hierin beschriebene Extrakt, mit 80%igem Ethanol als Auszugsmittel, konnte *invitro* gute Zellschutzeigenschaften demonstrieren. Hingegen offenbart diese Veröffentlichung sowohl einen toxischen Effekt einer Hyperforin-Fraktion, als auch einen protektiven Effekt der Flavonoid-Fraktionen. Johanniskrautextrakte sind als weltweit verwendeter Extrakt bei milden Depressionen auch hinsichtlich ihres Nebenwirkungspotentials gut untersucht. Gerade für Hyperforin und sehr Hyperforin hochgehaltige Extrakte sind allerdings die meisten Nebenwirkungsmeldungen belegt. [Johne et al. (2003) BGBL 46:1061-1067 / DOI 10.1007/s00103-003-0742-y] Beschrieben sind auch dosisabhängige Nebenwirkungen und Wechselwirkungen mit Cytochromoxidasen des P450-Systems [Moore et al.(2000) St.John's wort induces hepatic drug metabolism through activation of the pregnane X receptor.Proc Natl Acad Sci USA 97:7500-7502]. Diese Wechselwirkungen führen zu Interaktionen mit dem Metabolismus einer Reihe von zugelassenen Medikamenten. Sporadische dementielle Erkrankungen treten im Alter von über 60 Jahren auf, einem Zeitpunkt zu dem eine Vielzahl von Patienten bereits mit Medikamenten gegen Hyperlipidämie, Hypertonie oder anderen Auswirkungen des metabolischen Syndroms behandelt werden.

Das P450-Nebenwirkungsspektrum ist insbesondere auf die Menge an Hyperforin in den Extrakten zurückzuführen, während zu hohe Hypericin-Gehalte für phototoxische Nebenwirkungen verantwortlich gemacht werden.

Aufgabe der vorliegenden Erfindung war es, Mittel zur Verfügung zu stellen, die zur Behandlung von neurologischen Erkrankungen - degenerativer Art - geeignet sind und möglichst eine gute Wirksamkeit mit einem geringen Nebenwirkungspotential kombinieren.

Gelöst wird die Aufgabe durch die Verwendung von Pflanzenextrakten ausgewählt aus Gliederkräuterextrakten, erhältlich durch Extraktion mit Wasser oder wässrig-alkoholischen Lösungsmitteln oder Mischungen davon zur Verwendung in der Behandlung oder Prävention von neurodegenerativen Erkrankungen, wobei es sich bei der neurodegenerativen Erkrankung um eine Erkrankung handelt, welche mit akkumulierten Proteinen/Peptiden in Verbindung gebracht wird.

Gegenstand der Erfindung ist somit die Verwendung von Extrakten aus Pflanzen der Familie Lamioideae (Gliederkräutergewächse).

Aus den oberirdischen Teilen der Gliederkräuter werden mit wässrigen bzw. wässrig-alkoholischen Lösungsmitteln entsprechende Extrakte hergestellt.

Wässrig-alkoholische Lösungsmittel bedeutet, dass sie Mischungen von Wasser und Alkoholen darstellen. Bevorzugt liegt der Alkoholgehalt im Bereich von 10 bis 90%. Die Angaben hinsichtlich der Konzentration der Alkohole sind in % (V/V) bei 25°C angegeben.

Eine Extraktion mit wässrig-alkoholischen Lösungsmitteln bedeutet, dass die primäre Extraktion aus der Droge mit diesem Lösungsmittel durchgeführt wird.

Die entsprechenden Extraktzubereitungen eignen sich zur Behandlung oder Prävention von neurodegenerativen Erkrankungen. Ohne an diese Theorie gebunden zu sein, wird vermutet, dass die Wirksamkeit der Extrakte über eine Aktivierung der ABC-Transporter ablaufen könnte.

Aktuelle Veröffentlichungen der Antragsteller [Pahnke et al. 2008: Clinico-pathological function of cerebral ABC transporters - implications for the pathogenesis of Alzheimer's disease. Current Alzheimer Research 5, 396-406; Pahnke et al. 2009: Alzheimer's disease and blood-brain barrier function - Why have anti-β-amyloid therapies failed to prevent dementia progression? NeurosciBiobehavR 33:1099-1108] zeigen, dass ABC-Transporter eine wichtige Rolle für den Abtransport von Proteinablagerungen bei Demenzerkrankungen, insbesondere ß-Amyloidablagerungen bei der AD, spielen. Diese Transporter befinden sich an natürlichen Barrieren, wie z.B. der Blut-Hirn-Schranke. Eine Aktivierung dieser Transporter, von denen 49 humane Transporter bekannt sind, könnte zur Reduktion von intrazerebralen Proteinaggregaten und Peptidaggregaten führen.

Erfindungsgemäß ist es das Ziel, mittels Pflanzenextrakten bzw. Extraktkombinationen die pathologische Aggregation und Akkumulation von Proteinen *in vivo* zu verhindern bzw. bereits aggregierte Proteinansammlungen (auch Oligomere) teilweise oder vollständig aufzulösen und nachfolgend die Degradationsprodukte aus dem Gehirn/den Organen zu entfernen. Letztlich kann sowohl die Konzentration toxischer Intermediate, als auch die Aggregation in den Neuronen durch die erfindungsgemäße Verwendung von Pflanzenextrakten reduziert werden.

Erfindungsgemäß sollen die Pflanzenwirkstoffe dazu beitragen, sowohl intraals auch extraneuronale lösliche und unlösliche Proteinaggregate zu reduzieren. Da für viele dementielle Erkrankungen mit einer Dauertherapie bis ans Lebensende gerechnet werden muss, sollten für ein gutes Nutzen-Risiko-Verhältnis möglichst gut verträgliche, nebenwirkungsarme Substanzen verwendet werden. Aus der Therapie anderer Störungen und Erkrankungen des ZNS sind pflanzliche Extrakte hinlänglich für eine gute Verträglichkeit und Nebenwirkungsarmut bekannt.

Die Ergebnisse zeigen, dass es auch zu einer Verringerung der Plaquesanzahlen kommt.

In EP 1 054 682 wird eine *in-vitro*-Aktivität von Hyperforin in einem Lernleistungs-Modell beschrieben, welches mit Demenz in Verbindung gebracht wurde. Eigene Untersuchungen *in vivo* an Mausmodellen der AD zeigten jedoch überraschenderweise, dass die Aktivitäten der getesteten Johanniskrautextrakte keine Korrelation zum Hyperforingehalt aufwiesen (siehe Beispiele 2 und 3). Hyperforin-arme Extrakte sind jedoch besonders aktiv und mehr noch die gesamte Extraktmatrix - wesentlich bestimmt durch das Auszugsmittel im Herstellverfahren - begründet den Unterschied an Mausmodellen der AD (s. Tabelle Bsp. 7).

Bei den neurodegenerativen Erkrankungen handelt es sich um Erkrankungen welche mit akkumulierten Proteine oder Peptiden in Verbindung gebracht werden, sogenannte neurodegenerative Erkrankungen mit Proteinablagerungen.

Bei den Erkrankungen kann es sich um das Down-Syndrom, Chorea Huntington (HD), Spinozerebelläre Ataxien (SCA), insbesondere SCA2, oder Amyloidosen, insbesondere Morbus Alzheimer (AD), handeln.

Die erfindungsgemäßen Extrakte sind auch einsetzbar zur Behandlung von Entstehungsformen/Frühformen einer Demenzerkrankungen wie mild cognitive impairment (MCI) bei Morbus Alzheimer.

In einer Ausführungsform wird der Extrakt hergestellt aus Herba Sideritis spp. Für Sideritis-Extrakte haben sich insbesondere wässriges Solvens als auch wässrige Alkoholmischungen ausgewählt aus Methanol, Ethanol, 1-Propanol oder 2-Propanol in Mengen von 10 bis 70 Vol.-Anteilen Alkohol als bevorzugt erwiesen. Besonders bevorzugt sind Alkoholkonzentrationen zwischen 20 bis 40%-Vol. Alkohol.

Die erhaltenen Extrakte können in geeigneten Zubereitungen eingesetzt werden, insbesondere als Tabletten, Tinkturen, Kapseln, Sacchets, Trinkampullen oder Lutschtabletten.

Native Extraktmengen von 300 mg bis 1200 mg pro Tag sind besonders geeignet.

Die erfindungsgemäßen Extrakte können zur Herstellung eines Arzneimittels verwendet wird. Es kann sich allerdings auch um ein Lebensmittel oder ein Nahrungsergänzungsmittel oder eine ergänzende bilanzierte Diät handeln, die zur Unterstützung einer medikamentösen Behandlung der neurodegenerativen Erkrankung eingesetzt werden.

In einer weiteren Ausführungsform können als Gliederkräuterextrakte Herba Sideritis scardica, Herba Sideritis euboa oder Mischungen hiervon eingesetzt werden.

Die Erfindung wird durch die nachfolgenden Beispiele näher erläutert.

### Beispiel 1: Testmodell

Die Wirksamkeit der getesteten Extrakte wurde in folgendem in-vivo-Modell untersucht:

Ein genetisch generierter Mausstamm mit Alzheimer-Prädisposition (siehe z.B. Oakley H. et al. 2006, Intraneuronal beta-amyloid aggregates, neurodegeneration, and neuron loss in transgenic mice with five familial Alzheimer's disease mutations: potential factors in amyloid plaque formation. J Neurosci 26(40):10129-40) entwickelt in der Regel ab 1,5-2 Monaten nach der Geburt erste Ablagerungen des ß-Amyloid. Ab Tag 50 wird den Mäusen täglich eine Dosis des Extraktes aktiv geschlündelt. Nach einer definierten Gesamtlebenszeit von 75 bzw. 100 Tagen werden die Tiere getötet und das Gehirn analysiert. Hierzu wird molekularbiologisch mittels ELISA die Konzentration der löslichen Aß42 Fraktion gemessen. Histologisch werden die Plaque-Ablagerungen auf mehrere Parameter: Anzahl und Fläche untersucht (Mittelwerte; n≥3).

### Beispiel 2: Vergleichsbeispiel Johanniskraut 80% EtOH hyperforinarm

10,7 kg Johanniskraut (Ph.Eur.) werden bei 45°C zweimal mit 100 Litern Ethanol 80% (V/V) mazeriert. Das Eluat wird durch Filtration von Schwebstoffen befreit und schonend zu einem Dickextrakt aufkonzentriert. Der so erhaltene Spissum wurde bei 4°C kühl gestellt und nach 8 Stunden wurde eine oben aufsitzende Harzphase abgeschöpft. Der verbliebene Spissum wurde 70% nativ mit 14% Maltodextrin, 14% silikonisierter mikrokristalliner Cellulose, und mit 2% hochdispersem Siliciumdioxid getrocknet und gemahlen. Es resultieren 4,5 kg Extrakt, welcher durch einen Gehalt an Hypericin von 0,09%, an Hyperforin von 0,3% und an Flavonoiden von 5,7% gekennzeichnet war.

Der so erhaltene Extrakt wurde in dem in Beispiel 1 beschriebenen *in vivo-*Modell getestet. Die Ergebnisse sind in Beispiel 7 enthalten.

### Beispiel 3: Vergleichsbeispiel Johanniskraut 80% EtOH mit üblichem Hyperforingehalt

10 kg Johanniskraut (Ph.Eur.) werden bei 50°C mit 150 Litern Ethanol 80% (V/V) erschöpfend extrahiert. Das Eluat wird nach seiner Abkühlung auf Raumtemperatur durch Filtration von Schwebstoffen befreit und schonend zu einem Dickextrakt aufkonzentriert. Der so erhaltene Spissum wurde 98% nativ mit 2% hochdispersem Siliciumdioxid getrocknet und gemahlen. Es resultieren 2,2 kg Extrakt, welcher durch einen Gehalt an Hypericin von 0,21%, an Hyperforin von 2,9% und an Flavonoiden von 11,3% gekennzeichnet war.

Der so erhaltene Extrakt wurde in dem in Beispiel 1 beschriebenen *in vivo-*Modell getestet. Die Ergebnisse sind in Beispiel 7 enthalten.

### Beispiel 4: erfindungsgemäßes Beispiel aus Herba Sideritis scardica

10 kg Gliederkräuter (Sideritis scardica L.) wurden mit 300 Litern Ethanol 20% (V/V) bei 50°C erschöpfend perkoliert. Das Eluat wurde von der Droge abgelassen und von Drogenresten befreit. Das Perkolat wurde schonend im Unterdruck eingedampft und durch mehrmalige Wasserzugabe lösemittelfrei gefahren. Als Ausbeute resultierten 4,2 kg Dickextrakt mit einem Trockensubstanzanteil von 43,3%. Dieser wurde mit dem Trockenhilfsstoff Maltodextrin im Verhältnis 70% nativ: 30% Maltodextrin getrocknet.

Der so erhaltene Extrakt wurde in dem in Beispiel 1 beschriebenen *in vivo-*Modell getestet. Die Ergebnisse sind in Beispiel 7 enthalten.

### Beispiel 5: erfindungsgemäßes Beispiel aus Herba Sideritis euboa

10 kg Gliederkräuter (Sideritis euboa) wurden mit 300 Litern Ethanol 20% (V/V) bei 50°C erschöpfend perkoliert. Das Eluat wurde von der Droge abgelassen und von Drogenresten befreit. Das Perkolat wurde schonend im Unterdruck eingedampft und durch mehrmalige Wasserzugabe lösemittelfrei gefahren. Als Ausbeute resultierten 3,9 kg Dickextrakt mit einem Trockensubstanzanteil von 42,6%. Dieser wurde mit dem Trockenhilfsstoff Maltodextrin im Verhältnis 70% nativ: 30% Maltodextrin getrocknet.

Der so erhaltene Extrakt wurde in dem in Beispiel 1 beschriebenen *in vivo-*Modell getestet. Die Ergebnisse sind in Beispiel 7 enthalten.

### Beispiel 6: erfindungsgemäßes Beispiel aus Herba Sideritis spp.

10 kg Gliederkräuter (50/50 Mischung aus Sideritis scardica L. und S. euboa L. wurden mit 300 Litern Ethanol 20% (V/V) bei 50°C erschöpfend perkoliert. Das Eluat wurde von der Droge abgelassen und von Drogenresten befreit. Das Perkolat wurde schonend im Unterdruck eingedampft und durch mehrmalige Wasserzugabe lösemittelfrei gefahren. Als Ausbeute resultierten 3,2 kg Dickextrakt mit einem Trockensubstanzanteil von 56%. Dieser wurde mit dem Trockenhilfsstoff Maltodextrin im Verhältnis 70% nativ: 30% Maltodextrin getrocknet.

Der so erhaltene Extrakt wurde in dem in Beispiel 1 beschriebenen *in vivo-*Modell getestet. Die Ergebnisse sind in Beispiel 7 enthalten.

### Beispiel 7: Ergebnisse der β-Amyloid-Bildung im in vivo Test:

Der untersuchte Mäusestamm gemäß Beispiel 1 ist genetisch derart verändert, dass die Alzheimer-typische Amyloidablagerungen nach ca. 100 Lebenstagen ausgeprägt sind. Der Beginn der Behandlung erfolgte nach 50 Lebenstagen.

| | Menge der löslichen Fraktion Aß42 (Mittelwert in ng je mg Hirngesamtprotein) | Behandlungs-zeitraum in Tagen (Alter in Tagen bei Analyse) |
|---|---|---|
| Kontrollstamm (H₂O-Behandlung) | 54,1 | 25d (75d) |
| Hypericum 80 % EtOH Hyperforin-arm nach Beispiel 2 | 23,9 | 25d (75d) |
| Hypericum 80% EtOH Hyperforin-reich nach Beispiel 3 | 35,3 | 25d (75d) |
| Sideritis scardica nach Beispiel 4 | 20,3 | 25d (75d) |
| Sideritis euboa nach Beispiel 5 | 18,3 | 25d (75d) |
| Sideritis spp. nach Beispiel 6 | 32,8 | 25d (75d) |

Die Sideritis-Extrakte nach den Beispielen 4-6 senken die lösliche Fraktion des β-Amyloids gegenüber Kontrollbehandlung mit Wasser. Während S. scardica eine Reduzierung der löslichen Aß42-Fraktion um 62,5% bewirkte, konnte S. euboa eine Reduktion um 66,2% erreichen (n≥3). Damit konnte eine stärkere Reduzierung der löslichen Aß42-Fraktion erreicht werden, als sie mit den Johanniskrautextrakten aus EtOH 80% gewonnen wurden, die als literaturbekannter Hinweis (Silva, B. et al., Neurotoxicity Research 2004, 119-130) und somit Stand der Technik mit dem angewandten Testmodell bestätigt wurden.

Somit ist nicht ein Inhaltsstoff des Extraktes, sondern die gesamte Extraktmatrix - bestimmt durch die verwendete Pflanzenspecies, das Lösungsmittel im Herstellverfahren - das entscheidende Merkmal für die erfolgreiche Verwendung eines Sideritis-Extraktes im Bereich neurodegenerativer Erkrankungen.

### Beispiel 8 - Histologische Auswertung der Plaquebildung

Der untersuchte Mäusestamm gemäß Beispiel 1 ist genetisch derart verändert, dass die Alzheimer-typische Amyloidablagerungen nach ca. 100 Lebenstagen ausgeprägt sind. Der Beginn der Behandlung erfolgte nach 50 Lebenstagen.

Abhängigkeit der Plaque-Bildung nach Gabe von 4000 mg/kg Johanniskraut-Extrakt gemäß Beispiel 2 von der Behandlungsdauer (n≥5)

| Dauer | 75 d | 100 d |
|---|---|---|
| Plaque-Anzahl (N) | | |
| Kontrollgruppe | 109,6 | 203,0 |
| Johanniskraut-Extrakt nach Bsp. 2 | 64,5 | 129,5 |
| Relat. Veränderung | -41,1% | -36,2% |

| mittlere Plaque-Fläche (A in µm²) | | |
|---|---|---|
| Kontrollgruppe | 380,6 | 498,0 |
| Johanniskraut-Extrakt nach Bsp. 2 | 337,4 | 367,5 |
| Relat. Veränderung | -11,3% | -26,2 % |

### Beispiel 9 - Histologische Auswertung der Plaque-Bildung

Wie im Beispiel 8 wurde die Plaque-Bildung bei Gabe von Sideritis-Extrakten gemessen.

Veränderung der Plaque-Bildung nach Gabe von 4000 mg/kg KG Sideritis-Extrakt gemäß Beispiel 6 (n≥5):

| Dauer | 100 d |
|---|---|
| | Plaque-Anzahl (N) |
| Kontrollgruppe | 203,0 |
| Sideritis-Extrakt nach Bsp. 6 | 103,6 |
| Relat. Veränderung | -49% |
| | |
| | mittlere Plaque-Fläche (A in µm²) |
| Kontrollgruppe | 498,0 |
| Siderits-Extrakt nach Bsp. 6 | 422,2 |
| Relat. Veränderung | -15,2% |

Bei annähernd gleichbleibender Plaque-Fläche ist die Anzahl deutlich zurückgedrängt worden.

### Beispiel 10 - erfindungsgemäßes Beispiel aus Herba Sideritis scardica

10,1 kg Gliederkräuter (Sideritis scardica L.) wurden mit 384 Litern gereinigtem Wasser bei 80°C erschöpfend perkoliert. Das Eluat wurde von der Droge abgelassen und von Drogenresten mittels Filtration über einen Cellulosefilter (250 µm) befreit. Das klare Perkolat wurde schonend im Unterdruck eingedampft. Als Ausbeute resultierten 2,1 kg Dickextrakt mit einem Trockensubstanzanteil von 63,0%. Dieser wurde mit dem Trockenhilfsstoff Maltodextrin im Verhältnis 70% nativ: 30% Maltodextrin rezeptiert, pasteurisiert und per Vakuumtrocknung bei 55°C getrocknet. Der so erhaltene Extrakt wurde über ein 0,75 mm Sieb zu einem homogenen, braunen Pulver vermahlen.

Das wasserlösliche Pulver eignet sich zur Herstellung von Sachets, Instant-Trinkpulver, Brausetabletten oder als Zusatz in lösliche Getränke.

### Beispiel 11 - Tablettenformulierung

Eine Tablette enthält 300 mg Sideritis-Trockenextrakt gemäß Beispiel 6. Weitere Bestandteile sind Zerfallsbeschleuniger Natriumcarboxymethylcellulose, Fließhilfsmittel Siliciumdioxid, Bindemittel Polyethylenglykol 4000, Schmiermittel Magnesiumstearat, sowie Natriumhydrogencarbonat.

## Patentansprüche

1. Gliederkräuterextrakte erhältlich durch Extraktion mit Wasser oder wässrig-alkoholischen Lösungsmitteln zur Verwendung in der Behandlung oder Prävention von neurodegenerativen Erkrankungen, wobei es sich bei der neurodegenerativen Erkrankung um eine Erkrankung handelt, welche mit akkumulierten Proteinen/Peptiden in Verbindung gebracht wird.

2. Gliederkräuterextrakte zur Verwendung nach Anspruch 1, wobei es sich bei der neurodegenerativen Erkrankung um das Down-Syndrom, Chorea Huntington (HD), Spinozerebelläre Ataxien (SCA), insbesondere SCA2, oder Amyloidosen, insbesondere Morbus Alzheimer (AD), handelt.

3. Gliederkräuterextrakt zur Verwendung nach einem der vorhergehenden Ansprüche, wobei es sich um die Entstehungsform/Frühform einer Demenzerkrankung, wie mild cognitive impairment (MCI) bei Morbus Alzheimer, handelt.

4. Gliederkräuterextrakt zur Verwendung nach einem der Ansprüche 1 bis 3, wobei der Extrakt hergestellt wurde aus Herba Sideritis spp.

5. Gliederkräuterextrakt zur Verwendung nach einem der Ansprüche 1 bis 4, in Form eines Arzneimittels, eines Lebensmittels, eines Nahrungsergänzungsmittels oder einer ergänzenden bilanzierten Diät.

6. Gliederkräuterextrakt zur Verwendung nach einem der Ansprüche 1 bis 5, wobei zusätzlich ein Extrakt aus Johanniskraut enthalten ist, bevorzugt hergestellt mit 80%igem Ethanol als Auszugsmittel.

7. Gliederkräuterextrakt zur Verwendung nach einem der Ansprüche 1 bis 6, wobei als Gliederkräuter Herba Sideritis scardica, Herba Sideritis euboa oder Mischungen hiervon eingesetzt werden.

## Claims

1. Ironwort extracts obtainable by extraction with water or aqueous-alcoholic solvents for use in the treatment or prevention of neurodegenerative diseases, wherein said neurodegenerative disease is a disease related to accumulated proteins/peptides.

2. The ironwort extracts for use according to claim 1, wherein said neurodegenerative disease is Down syndrome, Huntington's disease (HD), spinocerebellar ataxia (SCA), especially SCA2, or amyloidoses, especially Alzheimer's disease (AD).

3. The ironwort extract for use according to any of the preceding claims, wherein said disease is an initial form/early form of a dementia disease, such as mild cognitive impairment (MCI) in Alzheimer's disease.

4. The ironwort extract for use according to any of claims 1 to 3, wherein said extract has been prepared from *Sideritis* spp. herb.

5. The ironwort extract for use according to any of claims 1 to 4 in the form of a medicament, food, food supplement or supplementary balanced diet.

6. The ironwort extract for use according to any of claims 1 to 5, additionally containing an extract from St. John's wort, preferably prepared with 80% ethanol as extractant.

7. The ironwort extract for use according to any of claims 1 to 6, wherein *Sideritis scardica* herb, *Sideritis euboa* herb or mixtures thereof are employed as said ironworts.

## Revendications

1. Extraits de gaillet jaune, pouvant être obtenus par extraction avec de l'eau ou des solvants aqueux-alcooliques, pour une utilisation dans le traitement ou la prévention de maladies neurodégénératives, dans lesquels il s'agit, en matière de maladie dégénérative, d'une maladie qui est mise en relation avec des protéines/peptides accumulés.

2. Extraits de gaillet jaune pour une utilisation selon la revendication 1, dans lesquels il s'agit, en matière de maladie dégénérative, du syndrome de Down, de la chorée de Huntington (HD), d'ataxies spinocérébelleuses (SCA), en particulier de SCA2, ou d'amyloïdoses, en particulier de la maladie d'Alzheimer (AD).

3. Extrait de gaillet jaune pour une utilisation selon une des revendications précédentes, dans lequel il s'agit de la forme naissante/forme précoce d'une maladie démentielle telle que le mild cognitive impairment (MCI) dans le cas de la maladie d'Alzheimer.

4. Extrait de gaillet jaune pour une utilisation selon une des revendications 1 à 3, dans lequel l'extrait est préparé à partir de Herba Sideritis spp.

5. Extrait de gaillet jaune pour une utilisation selon une des revendications 1 à 4, sous forme de médicament, d'aliment, de complément alimentaire ou de régime avec complément alimentaire équilibré.

6. Extrait de gaillet jaune pour une utilisation selon une des revendications 1 à 5, dans lequel un extrait de millepertuis est contenu en plus, préparé de préférence avec de l'éthanol à 80% en tant qu'agent d'extraction.

7. Extrait de gaillet jaune pour une utilisation selon une des revendications 1 à 6, dans lequel Herba Sideritis cardica, Herba Sideritis euboa ou des mélanges de ceux-ci sont utilisés en tant que gaillet jaune.
